(19) Europäisches Patentamt
European Patent Office
Office européen des brevets

(11) EP 0 335 972 B2

(12) NEW EUROPEAN PATENT SPECIFICATION

(45) Date of publication and mention of the opposition decision:
**18.06.1997 Bulletin 1997/25**

(45) Mention of the grant of the patent:
**03.03.1993 Bulletin 1993/09**

(21) Application number: **87907529.9**

(22) Date of filing: **13.11.1987**

(51) Int. Cl.$^6$: **A61L 33/00**

(86) International application number:
**PCT/JP87/00884**

(87) International publication number:
**WO 88/03814 (02.06.1988 Gazette 1988/12)**

# (54) MEDICAL MATERIAL AND PROCESS FOR ITS PRODUCTION

MEDIZINISCHES MATERIAL UND VERFAHREN ZUR HERSTELLUNG

MATERIAU MEDICAL ET PROCEDE DE PRODUCTION

(84) Designated Contracting States:
**BE DE FR GB IT NL**

(30) Priority: **21.11.1986 JP 278098/86**

(43) Date of publication of application:
**11.10.1989 Bulletin 1989/41**

(73) Proprietor: **TERUMO KABUSHIKI KAISHA**
**Tokyo 151 (JP)**

(72) Inventors:
- **KASHIWAGI, Nobuyoshi**
**Fuji-shi Shizuoka 417 (JP)**
- **SASAKI, Masatomi**
**Fuji-shi Shizuoka 417 (JP)**
- **MORITA, Hirotomo**
**Fuji-shi Shizuoka 417 (JP)**
- **SOENO, Rieko**
**Shibuya-ku Tokyo 151 (JP)**

(74) Representative: **Henkel, Feiler, Hänzel & Partner**
**Möhlstrasse 37**
**81675 München (DE)**

(56) References cited:

| | |
|---|---|
| EP-A- 0 103 816 | EP-A- 0 155 534 |
| JP-A- 80 461 | JP-A- 218 156 |

Remarks:
The file contains technical information submitted after the application was filed and not included in this specification



Printed by Rank Xerox (UK) Business Services
2.14.8/3.4

## Description

(Technical Field)

This invention relates to a medical material and a method for the production thereof. More particularly, it relates to a medical material capable of exhibiting high biocompatibility stably for a long time and a method for the production thereof.

(Background Art)

In recent years, artificial internal organs such as artificial kidneys, artificial livers, oxygenators, and blood separating devices have been produced and put to use. The biocompatibility of materials of which these artificial internal organs are made now poses an important problem. The term "biocompatibility" means that a given material, on contact with blood or vital tissues, should be incapable of injuring or fracturing blood cells in the blood or degenerating plasmatic proteins or inducing thrombosis. Since the biocompatibility of a medical material is a problem of the biochemistry which affects the surface of the material, the surface behavior of the material attracts the most careful attention. For the medical material to be safely used, it is only natural that the quality of the bulk of the material should deserve due respect as well. For the medical material, it is an essential requirement that the material should be endowed with proper surface quality and bulk quality. An examination of the surface quality and the bulk quality of a varying medical material reveals that after all they are mutually related independent factors and that simultaneous impartation of these independent factors to one material is obtained with great difficulty. The method which comprises selecting a material inherently possessing the bulk quality and altering only the surface quality of the material as required, therefore, may well be called an ideal approach to the problem under discusion because it is simplest chemically and safe medically.

From this point of view, various methods for surface modification have been developed. For example, the method for grafting a compound to the surface of a material has been applied to medical materials. Medical materials having polyacrylamide, N,N-dimethyl acrylamide, etc. grafted to their surfaces so far put to use are fulfilling their parts with fairly favorable results (Yoshito Ikada et al., "Glossary of Manuscripts for the Seventh Meeting of Japan Bio-Materials", November 1985). Since efforts are made to increase the chain length of a compound grafted to the surface of a medical material, the formation of a uniform graft is not easily obtained because the chains of the graft are heavily dispersed in length. Moreover, the graft component used for this purpose has not always imparted a fully satisfactory biocompatibility to the produced medical material. As means for graft treatment, such methods as the photo-graft polymerization and radiation graft polymerization have been known in the art. They impose great limits upon the shape of a material to be treated, the kind of substances used therefor, etc. and necessitate special devices. Thus, they do not easily find utility in a wade variety of applications.

The practice of grafting with a macromer has been recently disseminating. A graft copolymer is produced, for example, by mixing a macromer of methyl methacrylate with 2-hydroxyethyl methacrylate and polymerizing the resultant mixture. It is known that this graft copolymer exhibits a better ability to resist thrombosis than corresponding homopolymers and random copolymers ("Medical Materials and Living Organisms", 1st ed. 1982, page 37 and pages 287 to 289, compiled by Yukio Imanishi and published by Kodansha Scientific). In a graft copolymer obtained by the polymerization of a macromer with other monomer as described above, graft chains are formed not only on the surface of the polymer but also inside the bulk of the polymer. The materials, therefore, cannot be easily obtained with a necessary bulk quality.

The technique of imparting improved biocompatibility to a medical material by physically coating the surface of this medical material with a fat-soluble vitamin has been introduced to the art (Japanese Patent Laid-Open SHO 59(1984)-64,056). When an artificial internal organ having the surface thereof coated as described above with a fat-soluble vitamin is put to use, it induces secondary reactions very sparingly on living organisms and brings about substantially no transient leukopenia. Since the fat-soluble vitamin is deposited only physically on the surface, however, it exhibits a weak binding force and clearly liberates itself from the surface and passes into the blood. The vitamin coat, therefore, lacks stability.

An object of this invention, therefore, is to provide a novel medical material and a method for the production thereof.

Another object of this invention is to provide a medical material capable of exhibiting high biocompatibility stable for a long time and a method for the production thereof.

Still another object of this invention is to provide a medical material possessing a highly desirable bulk property and, at the same time, exhibiting a surface behavior rich in biocompatibility and a method for the production thereof.

A further object of this invention is to provide a medical material endowed on the entire surface thereof with graft chains of clear character and high mobility and a method for the production thereof.

(Disclosure of the Invention)

The objects described above are accomplished by a medical material characterized by possessing on the surface

of a substrate made of a polymer possessing hydroxyl-, amino- or carboxyl-group-containing (in the following designated as "functional group-containing") repeating units a graft polymer formed by causing a macromer possessing at one of the opposite terminals thereof a fat-soluble vitamin, a saturated fatty acid, or an unsaturated fatty acid residue to be bonded at the remaining reactive terminal thereof to the aforementioned hydroxyl-, amino- or carboxyl- group of the polymer existing on the surface of the substrate.

Preferably the functional group is a hydroxyl group.

This invention further comprises a medical material wherein the polymer is a regenerated cellulose or a cellulose derivative. This invention further discloses a medical material wherein the fat-soluble vitamin is selected from the group consisting of vitamin A, vitamin D, vitamin E, vitamin K, and ubiquinone, and preferably is vitamin E. This invention further discloses a medical material wherein the saturated fatty acid is selected from the group, consisting of lauric acid, myristic acid, pentadecylic acid, palmitic acid, and stearic acid, and preferably is myristic acid or palmitic acid. This invention further discloses a medical material wherein the unsaturated fatty acid is selected from the group consisting of elaidic acid, oleic acid, linolic acid, linolenic acid, arachidonic acid, and eicosapentaeic acid, and preferably is linolic acid or linolenic acid. This invention further discloses a medical material wherein the reactive terminal of the macromer is an epoxy group. This invention further discloses a medical material wherein the macromer is a linear diglycidyl ether derivative possessing at one of the opposite terminals thereof a fat-soluble vitamin, a saturated fatty acid, or an unsaturated fatty acid residue. This invention further discloses a medical material wherein the linear diglycidyl ether is 1,4-butane diol diglycidyl ether. This invention further discloses a medical material wherein the macromer is at least one alkylene glycol skeleton possessing at one of the opposite terminals thereof a fat-soluble vitamin, a saturated fatty acid, or an unsaturated fatty acid residue. This invention further discloses a medical material wherein the other reactive terminal is an epoxy group. This invention further discloses a medical material wherein the other reactive terminal is a hydroxyl group. This invention further discloses a medical material wherein the alkylene glycol has a polymerization degree in the range of 1 to 100. This invention further discloses a medical material wherein the alkylene glycol is polyethylene glycol or polypropylene glycol.

The objects described above are also accomplished by a method for the production of a medical material characterized by (a) forming a macromer possessing at one of the opposite terminals thereof a fat-soluble vitamin, a saturated fatty acid, or an unsaturated fatty acid residue, and (b) causing the other reactive terminal of the macromer to be bonded, on a substrate made of a polymer possessing functional group-containing repeating units, to the functional group of the polymer existing on the surface of the substrate.

This invention also discloses a method for the production of a medical material wherein the macromer to be formed possesses an epoxy group as the other reactive terminal. This invention further discloses a method for the production of a medical material wherein the macromer is formed by causing the functional group of a fat-soluble vitamin, a saturated fatty acid, or an unsaturated fatty acid to react selectively with the reactive group at one of the opposite terminal of a macromer precursor possessing reactive groups at the opposite terminals thereof. This invention further discloses a method for the production of a medical material wherein the macromer precursor is a diepoxide. This invention further discloses a method for the production of a medical material wherein the diepoxide is a linear diglycidyl ether. This invention further discloses a method for the production of a medical material wherein the linear diglycidyl ether is 1,4-butane diol diglycidyl ether. This invention further discloses a method for the production of a medical material wherein the macromer precursor possesses at least one alkylene glycol skeleton. This invention further discloses a method for the production of a medical material wherein the functional group of a fat-soluble vitamin, a saturated fatty acid, or an unsaturated fatty acid is a carboxyl group. This invention further discloses a method for the production of a medical material wherein the functional group of a fat-soluble vitamin is a hydroxyl group. This invention further discloses a method for the production of a medical material wherein the macromer is bonded to the surface of the substrate by causing the macromer to come into contact in a liquid state or a gaseous state with the surface of the substrate thereby allowing the other reactive terminal of the macromer to react with the functional group on the surface of the sbustrate. This invention further discloses a method for the production of a medical material wherein the macromer is bonded to the surface of a substrate possessing a functional OH group by causing the macromer to come into contact in a liquid state with the surface of the substrate in the presence of a Friedel-Crafts type catalyst or an alkali catalyst thereby allowing the reactive epoxy group terminal of the macromer to react with the functional OH group on the surface of the substrate. This invention further discloses a method for the production of a medical material wherein the Friedel-Crafts type catalyst is boron trifluoride. This invention further discloses a method for the production of a medical material wherein the alkali catalyst is sodium hydroxide or potassium hydroxide. This invention further discloses a method for the production of a medical material wherein the solvent is at least one member selected from the group consisting of dioxane, acetone and methylethyl ketone.

(Brief Description of the Drawings)

Fig. 1 is a partially cutaway perspective view illustrating the construction of a dialyzer using a medical material of the present invention,

Fig. 2 is a diagram illustrating an experimetal circuit for the extracorporeal circulation using the dialyzer, and

Fig. 3 is a graph showing the time-course change in the number of white blood cells.

(Best Mode for Carrying Out the Invention)

The medical material of the present invention is characterized by possessing on the surface of a substrate made of a polymer possessing functional group-containing repeating units a graft layer formed by causing a macromer possessing at one of the opposite terminals thereof a fat-soluble vitamin, a saturated fatty acid, or an unsaturated fatty acid residue to be bonded at the remaining reactive terminal thereof to the functional group of the polymer existing on the surface of the substrate. Since the medical material of this invention has graft chains formed only on the surface of the substrate thereof, it permits alteration of the surface behavior of the substrate without affecting the bulk property thereof. Since the graft chains are uniform and prominent, their length and mobility can be easily controlled. Further the fat-soluble vitamin, unsaturated fatty acid, or graft chains are not liberated and are rich in mobility, the medical material enjoys enhanced biocompatibility.

The term "macromer" generally means a polymer of the type possessing polymerizing functional groups at the terminals thereof. As used in the present specification, this term should be construed in a broader sense as meaning a macromolecular compound possessing reactive functional groups at the terminals thereof.

Now, the present invention will be described more specifically below with reference to embodiments thereof.

In the medical material of the present invention, the polymer which constitutes itself the substrate of the medical material may be any of the polymers which possess functional group-containing repeating units. It is suitably selected to fulfil the bulk property the produced medical material is expected to possess. Examples of the functional group include hydroxyl group, amino group, and carboxyl group. Examples of the polymer which possess a hydroxyl group as a functional group include regenerated cellulose and various cellulose derivatives. In the repeating unit of regenerated cellulose shown below, for example, the hydroxyl groups indicated by an asterisk (*) are functional groups. The hydroxyl group attached at 3 position which is not asterisked is deficient in reactivity with respect to the construction of cellulose.

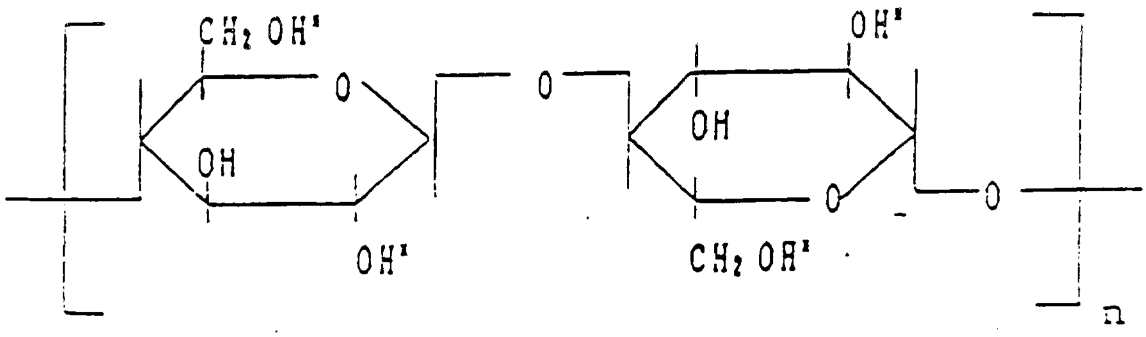

Examples of the fat-soluble vitamin usable in the present invention include vitamin A, vitamin D, vitamin E, vitamin K, and ubiquinone. Among other fat-soluble vitamins cited above, vitamin E proves to be particularly desirable.

Typical examples of the vitamin A include vitamins A such as retinol (vitamin $A_1$ alcohol), retinal (vitamin $A_1$ aldehyde), vitamin $A_1$ acid, 3-dehydroretinol (vitamin $A_2$ alcohol), and 3-dehydroretinal (vitamin $A_2$ aldehyde), provitamins A such as β -carotene, β ,β -carotene, α-carotene, β , ε-carotene, γ -carotene, and β- ψ - carotene, and cisvitamins A.

Typical examples of the vitamin D include vitamins D such as vitamin $D_2$, vitamin $D_3$, vitamin $D_4$, vitamin $D_5$, vitamin $D_6$, and vitamin $D_7$ and provitamins D analogous thereto.

Typical examples of the vitamin E include tocopherols such as α -tocopherol, β -tocopherol, γ - tocopherol, and δ - tocopherol and tocotrienols such as -tocotrienol. β-tocotrienol, γ -tocotrienol, and -tocotrienol.

Typical examples of the vitamin K include vitamin $K_1$ and vitamin $K_2$. Typical examples of the ubiquinone include ubiquinones 1 to 12 (Q-1 to Q-12) and oxides and amino analogs thereof.

Typical examples of the unsaturated fatty acid usable in the present invention include essential unsaturated fatty acids such as elaidic acid, oleic acid, linolic acid, linolenic acid, arachidonic acid, and eicosapentaeic acid. Among other unsaturated fatty acids cited above, linolic acid and linolenic acid prove to be particularly desirable.

Typical examples of the saturated fatty acid usable in the present invention include essential saturated fatty acids such as lauric acid, myristic acid, pentadylic acid, palmitic acid, and stearic acid. Among other saturated fatty acids cited above, myristic acid and palmitic acid prove to be particularly desirable.

The medical material of the present invention possesses on the surface of a substrate made of a polymer possessing repeating units containing such a functional group as described above a graft layer formed by causing a macromer

possessing at one of the opposite terminals thereof a fat-soluble vitamin, a saturated fatty acid, or an unsaturatd fatty acid residue to be bonded at the remaining reactive terminal thereof to the aforementioned terminal group of the polymer existing on the surface of the substrate.

The macromer possessing a fat-soluble vitamin, a saturated fatty acid, or an unsaturated fatty acid residue at one of the opposite terminals thereof can be formed with chains of a desired length. It should be designed so that the graft chains to be formed of the macromer will exhibit sufficient mobility and high stability and, consequently, the fat-soluble vitamin, saturated fatty acid, or unsaturated fatty acid bonded to the leading ends of the graft chains will impart high and stable biocompatibility to the produced macromer. The macromer skeleton, therefore, is desired to have a suitable chain length, for example, in the range of $1 \times 10^{-3}$ to $1 \times 10^{-1}$ μm, preferably $2 \times 10^{-3}$ to $5 \times 10^{-2}$ μm, and to form a flexible linear construction devoid of an annular configuration or a triple bond. The other reactive terminal of the macromer may be in any form on the sole condition that it should be capable of forming a chemical bond through reaction with the functional group such as, for example, hydroxyl group, amino group, or carboxyl group, on the surface of the substrate. Examples of the reactive terminal include epoxy group and hydroxyl group, preferably epoxy gorup.

Typical constructions of the macromer to be used in the present invention include those shown below, for example.

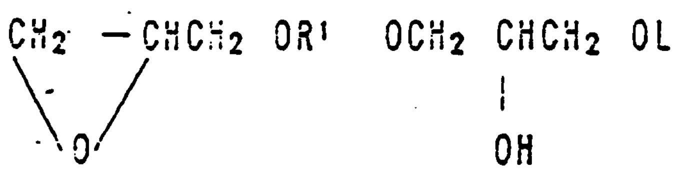

wherein $R^1$ is polymethylene having 2 to 200, preferably 4 to 100 carbon atoms and L is a fat-soluble vitamin, unsaturated fatty acid, or saturated fatty acid residue, and

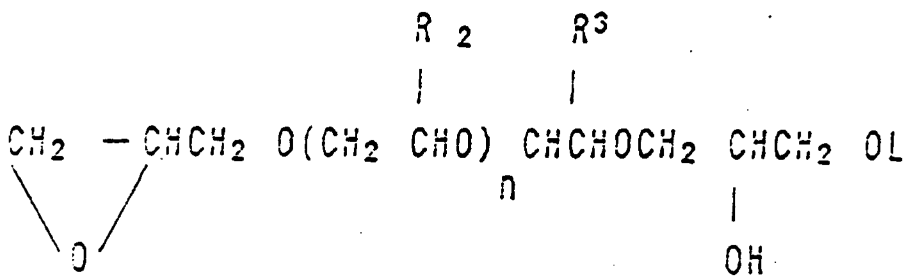

wherein $R^2$ and $R^3$ are independently hydrogen atom or alkyl group of 1 to 4 carbon atoms, L is a fat-soluble vitamin, unsaturated fatty acid, or saturated fatty acid residue, and n is a number in the range of 0 to 100, preferably 1 to 100, and more preferably 2 to 50.

The macromer described above can be formed by a varying method which suits the terminal group possessed by the fat-soluble vitamin, unsaturated fatty acid, or unsaturated fatty acid bonded to one of the opposite terminals of the macromer and the reactive terminal group introduced to the other terminal. The formation of a macromer which possesses at one of the opposite terminals thereof a compound possessing a terminal OH group selected from among such fat-soluble vitamins as retinol, 3-dehydroretinol, vitamin $D_2$, vitamin $D_4$, vitamin $D_5$, vitamin $D_6$, vitamin $D_7$ and provitamins D analogous thereto, α-tocopherol, β-tocopherol, γ -tocopherol, δ-tocopherol, α-tocotrienol, β-tocotrienol, γ-tocotrienol, and δ -tocotrienol or a compound residue possessing terminal COOH group selected from among fat-soluble vitamins such as vitamin $A_1$ acid and unsaturated fatty acids and saturated fatty acids mentioned above and possessing at the other reactive terminal an epoxy group, for example, is accomplished advantageously by using a diepoxide such as a linear diglycidyl ether as a macromer precursor. Specifically, this formation is attained by causing the reactive terminal group (such as, for example, OH group or COOH group) of the fat-soluble vitamin, unsaturated fatty acid, or saturated fatty acid possessing an epoxy group and the reactive group mentioned above to be selectively bonded through reaction to only one of the epoxy groups of the diepoxide. The conditions for this selective reaction of the diepoxide may be found by the technique of organic synthetic chemistry. When a macromer possessing a vitamin E residue at one terminal is to be formed by using 1,4-butane diol diglycidyl ehter as a diepoxide, for example, it has been found that the macromer is produced in a high yield by causing 1 to 5 mols, more desirably 1 to 3 mols, most desirably about 2 mols, of the diglycidyl ether to react with 1 mol of vitamin E in dioxane as a solvent in the presence of 0.04 to 2 mols, more desirably 0.2 to 1.5 mols, and most desirably 1.2 mols, of a base such as NaH (sodium hydride) in an

atmosphere of nitrogen at temperature in the range of normal room temperature to 100° C, more desirably room temperature to 60° C, and most desirably about 40° C for a period in the range of 1 to 24 hours, more desirably 6 to 12 hours, and most desirably about 7 hours. When a macromer possessing at one terminal thereof an essential fatty acid residue such as of linolic acid, linolenic acid, or palmitic acid is to be formed by using 1,4-butane diol diglycideyl ether, it has been found that this macromer is produced in a high yield by causing 1 to 5 mols, more desirably 2 to 4 mols, and most desirably about 3 mols of diglycidyl ether to react with 1 mol of the essential fatty acid in dioxane as a solvent in the presence of 0.01 to 1 mol, more desirably 0.1 to 0.5 mol, and most desirably about 0.1 mol, of such an organic base as pyridine at a temperature in the range of normal room temperature to 100° C, more desirably 60° to 100° C, and most desirably about 80° C for a period in the range of 1 to 24 hours, more desirably 2 to 12 hours, and most desirably 7 to 8 hours. In either case, the isolation of the produced macromer for purification is advantageously attained by neutralizing the reaction mixture, extracting the neutralized reaction product from n-hexane, and subjecting the extract to sequential column chromatography.

When the macromer comprises an alkylene glycol skeleton and has bonded to one of the opposite terminals thereof a fat-soluble vitamin, unsaturated fatty acid, or saturated fatty acid residue similarly possessing a terminal OH group or terminal COOH group, for example, it can be formed by the method which is employed for the production of an alkylene glycol monoether or alkylene glycol monoester. The macromer possessing an alkylene glycol skeleton, for example, is obtained by causing ethylene oxide to react with the aforementioned fat-soluble vitamin, unsaturated fatty acid, or unsaturated fatty acid in the presence of an alkali catalyst under suitable temperature conditions or, where the macromer possesses a terminal COOH group, esterifying the fat-soluble vitamin, unsaturated fatty acid, or saturated fatty acid with alkylene glycol thereby forming an alkylene glycol skeleton having the fat-soluble vitamin, unsaturated fatty acid, or saturated fatty acid residue bonded to the one terminal thereof, and introducing a desired reactive terminal to the other terminal (OH group) of the alkylene glycol (not treatment whatever is required where the desired reactive terminal happens to be a hydroxyl group). Where an epoxy group is to be introduced as a reactive terminal, this introduction is advantageously effected by causing epichlorohydrin to react with the terminal OH group thereby forming glycidyl ether.

Of course, a macromer possessing an alkylene glycol skeleton and possessing an epoxy group as a reactive terminal can be formed by preparing a polyalkylene glycol type diglycidyl ether as a macromer precursor and causing a fat-soluble vitamin, unsaturated fatty acid, or saturated fatty acid to react selectivly with the epoxy group at one terminal as described above.

Where the macromer happens to possess an alkylene glycol skeleton, the degree of polymerization, though variable with the kind of alkylene, is desired to fall approximately in the range of 1 to 100. The alkylene glycol is particularly desired to be polyethylene glycol or polypropylene glycol. It is especially desirable to be a polyethylene glycol having a polymerization degree in the range of 20 to 70 or a polypropylene glycol having a polymerization degree in the range of 10 to 50.

The grafting of the macromer possessing the fat-soluble vitamin, unsaturated fatty acid, or saturated fatty acid residue at one terminal thereof as described above to the surface of a substrate made of a polymer possessing functional group-containing repeating units is accomplished by causing the reactive terminal of the macromer to be bonded by reaction to the functional group of the polymer present on the surface of the substrate. This reaction is accomplished by selecting reaction conditions suitable for the kind of the reactive terminal of the macromer and the kind of the functional group of the polymer forming the substrate and causing the macromer to coma into contact in a liquid state or a gaseous state with the surface of the substrate. Where the functional group of the polymer is an OH group and the reactive terminal of the macromer is an epoxy group, for example, the reaction for the bonding is attained by causing the macromer to come into contact with the surface of the substrate in the presence of a Friedel-Crafts type catalyst such as boron trifluoride, tin tetrachloride, or zinc chloride or an alkali catalyst at a suitable temperature. While the conditions for the reaction may be found in accordance with the knowledge of organic synthetic chemistry, the catalyst, the solvent, etc. which participate in the reaction must be selected with due consideration paid to the physiological safety of the produced raw material. To cite a typical set of desirable reaction conditions, the grafting of a macromer made of a diglycidyl ether derivative having a fat-soluble vitamin, unsaturated fatty acid, or saturated fatty acid bonded to one terminal thereof to the surface of a substrate made of regenerated cellulose, for example, is accomplished with desirable results by (A) (1) immersing the substrate in an aqueous solution of 0.1 to 10.0 w/v%, more desirably 0.2 to 5 w/v %, and most desirably 0.5 to 1.0 w/v %, of NaOH or KOH for a period in the range of 3 to 180 minutes, more desirably 10 to 60 minutes, and most desirably about 30 minutes at a temperature in the range of normal room temperature to 100° C, more desirably room temperature to 60° C, most desirably room temperature thereby converting the substrate into an alkali cellulose, (2) separately preparing a solution of 0.01 to 5 w/v %, more desirably 0.1 to 1.0 w/v %, and most desirably about 0.5 w/v % of the macromer in dioxane as a solvent, (3) removing the substrate of (1) and immersing it in the macromer solution of (2) and allowing the macromer to react with the substrate at a temperature in the range of normal room temperature to 40° C, preferably it room temperature for a period in the range of 6 to 48 hours, preferably 24 hours or at a temperature in the range of 60° to 100° C, preferably about 80° C for a period in the range of 2 to 10 hours, preferably 5.5 hours, and (4) finally removing the substrate from the solution and thoroughly washing it and (B) (1) preparing

a solution containing 0.01 to 5 w/v %, more preferably 0.1 to 1.0 w/v %, and most preferably about 0.5 w/v % of the aforementioned macromer and 0.01 to 1.0 w/v %, more preferably 0.02 to 0.5 w/v %, and most preferably 0.05 to 1.0 w/v %,of boron trifluoride in dioxane as a solvent, (2) immersing the substrate in the solution of (1) and allowing the macromer to react with the substrate at a temperature in the range of normal room temperature to 40° C, preferably room temperautre for a period in the range of 6 to 48 hours, preferably 24 hours or at a temperature in the range of 60° to 100° C, preferably about 80° C for a period in the range of 2 to 10 hours, preferably 5.5 hours, and (3) finally removing the substrate from the solution and thoroughly washing it. When sodium hydroxide, potassium hydroxide, or boron trifluoride is used as the catalyst, the washing of the reaction product is easily obtained and the produced raw materials warrants high safety on living organisms.

Now, the present invention will be described more specifically below with reference to several working examples, which are intended to be purely illustrative, not limitative in any sense, of the invention.

Example 1 Synthesis of linolic acid macromer

A mixture was obtained by adding 0.2373 g (0.0030 mol) of pyridine to 30 ml of dry dioxane. Then, 8.415 g (0.030 mol) of linolic acid was washed with the mixture into a four-neck flask having an inner volume of 200 ml under a current of nitrogen gas and stirred therewith at 80° C in the flask for 30 minutes. In the flask, 18.2052 g (0.090 mol) of 1,4-butane diol diglycidyl ether (1,4-BDGE) washed down therein with 20 ml of dry dioxane was stirred with the contents of the flask to induce reaction at 80° C for seven hours. This reaction was followed by sampling the reaction solution in a unit volume of 50μl, mixing the sample with 10 ml of a 9 : 1 n-hexane/1-propanol mixed solvent, and subjecting a 5 μl portion of the resultant solution to high-speed liquid chromatography (HPLC). In the HPLC, the sample solution was passed at a flow rate of 1 ml/min. through a column of Silica Nucleosil 50-5® measuring 4.6 mm in diameter and 300 mm in length, with the detection wavelength fixed at 210 nm. After the reaction was completed, the reaction solution and 20 ml of distilled water were homogeneously mixed and the resultant homogeneous mixture was extracted three times from 50 ml of n-hexane. Then, the n-hexane layer was dehydrated with anhydrous sodium sulfate, filtered, subjected to evaporation for expulsion of n-hexane, and subsequently left standing under a vacuum for expulsion of dioxane. The product thus product was refined through two stages of column chromatography. In the first stage, a column of Wako Gel C-200® measuring 27 mm in diameter and 300 mm in length and a mobile phase of 5 : 5 n-hexane/ethyl acetate were used and the solution was passed therethrough at a flow rate of 2 to 3 ml/min., with the detection wavelength fixed at 210 nm. In the second stage, a column of Wako Gel C-300® measuring 27 mm in diameter and 300 mm in length and a mobile phase of 5 : 5 n-hexane/ethyl acetate were used and the solution was passed therethrough at a flow rate of 0.5 to 1 ml/min. with the detection wavelength fixed at 210 nm. At the end of the purification, 11.1 g of refined linolic acid macromer having a purity of 85% was obtained in yield of 65%. The construction of the product was confirmed by NMR and IR spectrometry. By mass analysis, this product was identified to be refined linolic acid macromer having a molecular weight of 482.

Example 2 Synthesis of linolenic acid macromer

A linolenic acid macromer was synthesized by substantially following the procedure of Example 1 used or the synthesis of linolic acid macromer. A mixture was prepared by adding 0.2373 g (0.0030 mol) of pyridine to 30 ml of dry dioxane. Then, 8.3532 g (0.030 mol) of linolenic acid was washed with the mixture into a four-neck flask having an inner volume of 200 ml under a current of nitrogen gas and stirred at 80° C for 30 minutes. In this flask, 18.2052 g (0.090 mol) of 1,4-BDGE added thereto with 20 ml of dry dioxane were stirred with the contents of the flask at 80° C for eight hours to effect reaction. This reaction was followed by sampling the reaction mixture in a fixed volume of 50μl, mixing the sample with 10 ml of a 9 : 1 n-hexane/i-propanol mixed solvent, and analyzing a 4μl portion of the resultant solution to HPLC. In the HPLC, a column of Silica Nucleosil 50-5® measuring 4.6 mm in diameter and 300 mm in length and the solution was passed at a flow rate of 1 ml/min. with the detection wavelength fixed at 215 nm. After the reaction was completed, the reaction solution and 20 ml of distilled water were homogeneously mixed and the resultant mixture was extracted three times from 50 ml of n-hexane. Then, the n-hexane layer was dehydrated with anhydrous sodium sulfate, filtered, subjected to evaporation for expulsion of n-hexane, and left standing under a vacuum for expulsion of dioxane. Then, the product was refined by column chromatography. In the column chromatography, a column of Wako Gel C-300® measuring 25 mm in diameter and 300 mm in length and a mobile phase of a 5 : 5 n-hexane/ethyl acetate mixed solvent were used and the solution was passed therethrough at a flow rate of 0.5 to 1 ml/min., with the detection wavelength fixed at 210 nm. As the result, 10.2 g of refined linolenic acid macromer having a purity of 82% was obtained in a yield of 58%. The construction of this product was confirmed by NMR and IR spectrometry. By mass analysis, the product was identified to be refined linolenic acid macromer having a molecular weight of 480.

Example 3 Synthesis of palmitic acid macromer

A palmitic acid macromer was synthesized by substantially following the procedure of Example 1 used for the synthesis of linolic acid macromer. A mixture was prepared by adding 0.0090 mol (0.78 g) of pyridine to 90 ml of dry dioxane. Then, 0. 0090 mol (23.0675 g) of palmitic acid was washed with the aforementioned mixture into a 4-neck flask having an inner volume of 500 ml and stirred at 80° C for 30 minutes. In the flask, 0.270 mol (54.22 g) of 1,4-BDGE washed therein with 60 ml of dry dioxane was stirred with the contents of the flask at 90° C for eight hours to effect reaction. This reaction was followed by sampling the reaction solution and subjecting the sample to HPLC in the same manner as in Example 1. After the reaction was completed, the reaftion solution was combined with 150 ml of n-hexane and further with 40 ml of distilled water. Then, the n-hexane layer consequently formed was separated, subjected to evaporation for expulsion of n-hexane, and then left standing under a vacuum to effect expulsion of dioxane. The residue was combined with petroleum ether, cooled at 10° C overnight, and reprecipitated for separation. This product was refined by column chromatography in the same manner as in Example 1. Consequently, 25.6 g of refined palmitic acid macromer having a purity of 71% was obtained in yield of 41%. The construction of the product was confirmed by NMR and IR spectrometry. By mass analysis, the product was identified to be a refined palmitic acid macromer having a molecular weight of 458.

Example 4 Synthesis of vitamin E macromer

In a four-neck flask having an inner volume of 300 ml, (13.00 g) 0.03 mol of α-tocopherol and (12.06 g) 0.06 mol of 1,4-BDGE both dissolved in 90 ml of dry dioxane were left reacting in the presence of 0.036 mol (0.86 g) of NaH under a current of nitrogen at 40° C for seven hours. This reaction was followed by sampling the reaction solution in a fixed amount of 50μl and analyzing the same by HPLC. In the HPLC, the sample was passed through a column of Nucleosil 50-5® measuring 6 mm in diameter and 300 mm in length at a flow rate of 1 ml/min., with the detection wavelength fixed at 290 nm. After the reaction was completed, the reaction solution was added to 4 ml of dioxane and neutralized by addition of 1 ml of an aqueous 0.15% ammonium chloride solution, extracted from 5 ml of n-hexane, dehydrated with anhydrous sodium sulfate, filtered, neutralized by addition of 38.5 ml of an aqueous 7% ammonium chloride solution, and extracted three times from 50 ml of n-hexane. Then, the n-hexane layer was dehydrated with anhydrous sodium sulfate, filtered, subjected to evaporation for expulsion of n-hexane and then left standing under a vacuum to effect expulsion of dioxane. The product consequently obtained was refined further by column chromatography. In the column chromatography, the solution was passed at a flow rate of 2 to 3 ml/min. through a column of Wako Gel C-200® measuring 30 mm in diameter and 500 mm in length and a mobile phase of a 6 : 3 : 1 n-hexane/ethyl acetate/benzene mixed solvent, with the detection wavelength fixed at 280 nm. Consequently, 10.9 g of refined vitamin E macromer having a purity of 92% was obtained in a yield of 53%. The construction of this product was confirmed by NMR and IR spectrometry. By mass analysis, this compound was identified to be vitamin E macromer having a molecular weight of 633.

Example 5 Grafting of linolic acid macromer to regenerated cellulose

To the surface of a regenerated cellulose membrane, the linolic acid macromer obtained in Example 1 was grafted as follows. First, 0.3 g of a regenerated cellulose membrane (0.2 mm in thickness) was immersed for 30 minutes in 100 ml of an aqueous 0.5, 1.0, or 5.0 w/v % NaOH solution. Then, in the dioxane solution of 0.5 w/v % of linolic acid macromer obtained in Example 1, the cellulose membrane was immersed and left reacting therein at normal room temperature for 24 hours (or at 80° C for 5.5 hours). Then, the cellulose membrane was removed from the solution and thoroughly washed with distilled water to be used as a test piece.

Example 6 Grafting of linoleinic acid macromer to regenerated cellulose membrane

To the surface of a regenerated cellulose membrane, the linolenic acid macromer obtained in Example 2 was grafted as follows. First, in 100 ml of an aqueous 0.1, 0.5, 1.0 , or 5.0 w/v % NaOH solution, 0.3 g of a regenerated cellulose membrane (0.2 mm in thickness) was immersed for 30 minutes. Then, in the dioxane solution of 0.5 w/v % linoleinic acid macromer obtained in Example 2 was immersed and left reacting at room temperature for 24 hours (or at 80° C for 5.5 hours). Then, the cellulose membrane was removed from the solution and thoroughly washed with distilled water to be used as a test piece.

Example 7 Grafting of palmitic acid macromer to regenerated cellulose membrane

To the surface of a regeneratd cellulose membrane, the palmitic acid macromer obtained in Example 3 was grafted as follows. First, 0.3 g of a regenerated cellulose membrane (0.2 mm in thickness) was immersed for 30 min. in 100 ml of an aqueous 0.5, 1.0, or 5.0 w/v % NaOH solution. Then, in 100 ml of the dioxane solution of 0.5 w/v palmitic acid

macromer obtained in Example 3 was immersed and left reacting at room temperature for 24 hours (or at 80° for 5.5 hours). Then, the cellulose membrane was removed from the solution and thoroughly washed with dioxane, ethanol, and distilled water to be used as a test piece.

Example 8 Grafting (I) of vitamin E macromer to regenerated cellulose membrane

To the surface of a regenerated cellulose membrane, the vitamin E macromer obtained in Example 4 was grafted as follows. First, 0.3 g of a regenerated cellulose membrane (0.2 mm in thickness) was immersed for 30 minutes in 100 ml of an aqueous 1.0, 2.0, or 5.0 w/v % NaOH solution. Then, in 100 ml of the dioxane solution of 0.5 w/v % of vitamin E macromer obtained in Example 4, the cellulose membrane was immersed and left reacting at room temperature for 24 hours (or at 80° C for 5.5 hours). Then, the cellulose membrane was removed from the solution and thoroughly washed with dioxane, ethanol, and distilled water to be used as a test piece.

Example 9 Grafting (II) of vitamin E macromer to regenerated cellulose membrane

To the surface of a regenerated cellulose membrane, the vitamin E macromer obtained in Example 4 was grafted as follows. First, a solution containing in dioxane 0.5 w/v% of the vitamin E macromer obtained in Example 4 and 0.01, 0.1 or 1.0 w/v % of boron trifluoride was obtained. In 100 ml of this solution, 0.3 g of a regenerated cellulose membrane (0.2 mm in thickness) was immersed for 30 minutes and then left reacting therein at room temperature for 24 hours (or at 80° C for 5.5 hours). Then, the cellulose membrane was removed from the solution and thoroughly washed with dioxane, ethanol, and distilled water to be used as a test piece.

Example 10 Synthesis of polyethylene glycol diglycidyl ether

In a four-neck flask having an inner volume of 300 ml, 49.25 g (0.05 mol) of dry polyethylene glycol (having a molecular weight of 985) was homogeneously dissolved in 110 ml of dry dioxane and the resultant solution was combined with 0.0852 g of boron trifluoride diethyl ether complex salt, $BF_3O(C_2H_5)_2$. Then, the mixture was heated at 60° C and homogeneously stirred and 12.05 g (0.13 mol) of epichlorohydrin was added dropwise to the stirred mixture over a period of 30 minutes. Thereafter, the reaction was continued for two hours, with the reaction followed by gas chromatography under the following conditions. The reaction ratio was 97.9%. In the gas chromatography, a glass column measuring 3 mm in diameter and 200 mm in length and packed to capacity with PEG 600/Chromosorb W® was used and helium gas was passed as a carrier gas at a flow rate of 40 ml/min, with the column temperature fixed at 100° C.

Then, the reaction mixture was combined with 0.45 g of tetrabutyl ammonium bromide and kept at 60° C and 14.4 g (0.11 mol) of an aqueous 30 w/v% sodium hydroxide solution was added dropwise thereto over a period of 15 minutes and the resultant mixture was kept at 60° C for four hours to effect a reaction for removal of hydrochloric acid.

The resultant reaction composition was suction filtered through a glass filter, left standing under a vacuum for expulsion of the solvent, combined with 100 ml of chloroform, and washed twice with 50 ml of an aqueous 0.002M sodium dihydrogen phosphate solution. The washed reaction composition was dehydrated with anhydrous sodium sulfate and was vacuum dried for six hours. Consequently, there was obtained a polyethylene glycol diglycidyl ether having an epoxy equivalent of 595 (g/equivalent) in a yield of 74.4%. The theoretical epoxy equivalent is 549 (g/equivalent).

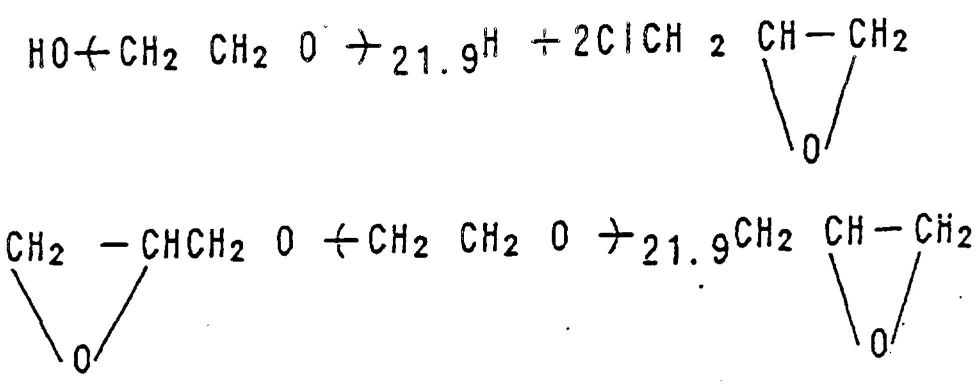

Example 11 Synthesis of linolic acid-polyethylene glycol diglycidyl ether macromer

In a four-neck flask having an inner volume of 200 ml, 7.5 ml of dry dioxane and 0.611 g of pyridine were placed and 2.1094 g (0.0075 mol) of linolic acid was added thereto under a current of nitrogen gas and they were stirred at 100° C for 30 minutes. The resultant mixture and 16.65 g (0.014 mol) of the polyethylene glycol diglycidyl ether obtained in Example 10 added thereto with 30 ml of dry dioxane were stirred at 100° C for reaction. This reaction was followed by sampling the reaction solution in a fixed amount of 50μl, dissolving the sample in 2 ml of tetrahydrofuran, and subjecting 10 μl of the resultant solution to HPLC. The reaction was terminated when the reaction ratio reached 94.4% (the reaction time amounting to five hours). In the HPLC, the solution was passed at a flow rate of 1 ml/min. through a column formed by connection of Shodex GPC KF-801® to Shodex GPC KF-802®, with tetrahydrofuran used as an eluate. The detection was performed with an ultraviolet spectrometer (detection wavelength at 210 nm) and a differential refractometer. After the reaction was completed, the reaction composition was left standing under a vacuum to expel dioxane, combined with 200 ml of hexane, thoroughly stirred, and centrifuged at 3,000 r.p.m. for 10 minutes for removal of the supernatant. The procedure just described was repeated once more. The sediment consequently obtained was combined with 120 ml of ether, stirred thoroughly, and centrifuged at 3,000 r.p.m. for 10 minutes. The supernatant was transferred to a separate centrifugal tube. The sediment was extracted from ether once more. The two ether extracts were combined. The combined extract was cooled with water until occurrence of precipitation. When the precipitate formed, it was subjected to cool at 0° C and to centrifuge at 3,000 r.p.m. The precipitate was vacuum dried and analyzed for epoxy equivalent, and subjected to IR measurement, NMR measurement, and HPLC analysis to confirm that the product aimed at was formed. The yield was 35.4%.

Example 12 Grafting (I) of linolic acid macromer to regenerated cellulose membrane

To the surface of a regenerated cellulose membrane, the linolic acid macromer obtained in Example 11 was grafted as follows. First, 0.3 g of a regenerated cellulose (0.2 mm in thickness) was immersed for 30 minutes in 100 ml of an aqueous 0.5 or 1.0 w/v % NaOH solution. Then, in the dioxane solution of 0.5 w/v % linolic acid macromer obtained in Example 1, the cellulose membrane was immersed and left reacting therein at room temperature for 24 hours (or at 80° C for 5.5. hours). Subsequently, the cellulose membrane was removed from the solution and thoroughly washed with dioxane, ethanol, and distilled water to be used as a test piece.

Example 13 Grafting (II) of linolic acid macromer to regenerated cellulose membrane

To the surface of a regenerated cellulose membrane, the linolic acid macromer obtained in Example 11 was grafted as follows. First, a solution containing 0.5 w/v % of the linolic acid macromer obtained in Example 11 and 0.1 w/v % of boron trifluoride in dioxane was prepared. In 100 ml of this solution, 0.3 g of a regenerated cellulose membrane (0.2 mm in thickness) was immersed for 30 minutes and left reacting at room temperature for 24 hours (or at 80 °C for 5.5 hours). Thereafter, the cellulose membrane was removed from the solution and thoroughly washed with dioxane, ethanol, and distilled water to be used as a test piece.

Evaluation Test 1: Test for ability to spread platelets

In a syringe made of polypropylene and containing 3.8% sodium citrate (1/10 of the volume of blood to be collected), the venous blood of a healthy man was collected. The blood mixture in the syringe was transferred into a test tube made of polypropylene so gently as to flow naturally down along the inner wall surface of the tube. The blood mixture in the tube was centrifuged at 800 r.p.m. for five minutes. The supernatant platelet-rich plasma (PRP) formed in the tube was collected and diluted with a 3.8% sodium citrate dilluent (1/10 in volume relative to Ringer's solution of lactic acid) to prepare a platelet suspension. The number of platelets in the platelet suspension was 66,000 per $mm^2$.

On the test pieces (1 $cm^2$) of the macromer-treated regenerated cellulose membranes obtained in Examples 5 to 9 and Examples 12 and 13 and on a test piece (1 $cm^2$) of untreated regenerated cellulose as a control, 0.2-ml portions of the platelet suspension were mounted in a thickness of 2 mm and left standing in contact with the test pieces at room temperature for 30 minutes. After the standing, the test pieces were washed lightly with the 3.8% sodium citrate diluent and were then placed in a 25% glutaraldehyde/Ringer's solution of lactic acid and left standing in a cool place overnight to be set. At the end of the this standing, the test pieces were lightly washed with the 3.8% sodium citrate diluent and dehydrated stepwise in ethanol series (sequentially treated with 50%, 60%, 70%, 80%, 90%, 95%, and 100% ethanol solutions each for 10 minutes). The test pieces were dried in draft and observed under a scanning electron microscope (produced by Japan Electron Optics Laboratory Co., Ltd. and marketed under produce code of "JSM-840"). They were evaluated with respect to the number of platelets deposited fast on a given test piece per 0.07 $mm^2$ and the morphological change. The morphological changes were classified under the following three types.

Type I: The platelets in the normal shape of discs were transformed into spheres each issuing force three to four pseudopodia, suggesting relatively weak adhesion of the platelets to the surface of raw material.

Type II: The platelets each issued forth more than several pseudopodia and spreading cells as far as one half the length of pseudopodium, suggesting strong adhesion of the platelets to the surface of raw material.

Type III: The platelets spread thin cells as far as more than half the length of pseudopodium and so fully as to assume the shape of substantial circles, suggesting perfect adhesion of the platelets to the surface of raw material.

The results of the test are shown in Table 1.

For the test pieces of Examples 5 to 9 and Examples 12 and 13, platelet suspensions originating in different individuals were used. Thus, test pieces of the untreated regenerated cellulose membrane as control were assigned one each to the group of test pieces of Examples 5 to 9 and the group of test pieces of Examples 12 and 13.

Table 1

| Test piece | | Macromer | Catalyst (concentration) | Reaction temperature | Form of platelet (%) Type I | Type II | Type III | Number of platelets deposited/0.07 mm$^2$ |
|---|---|---|---|---|---|---|---|---|
| Example 5 | A | linolic acid | NaOH (0.5%) | room temperature | 54.6 | 43.6 | 1.8 | 55 |
| | B | " | " (1.0%) | " | 94.4 | 5.6 | 0 | 36 |
| | C | " | " (5.0%) | " | 54.9 | 43.6 | 1.6 | 62 |
| Example 6 | A | linolenic acid | NaOH (0.1%) | room temperature | 66.8 | 32.7 | 0.5 | 119 |
| | B | " | " (0.5%) | " | 93.9 | 3.0 | 3.0 | 33 |
| | C | " | " (1.0%) | " | 70.3 | 28.3 | 1.4 | 188 |
| | D | " | " (5.0%) | " | 88.4 | 11.5 | 0 | 52 |
| Example 7 | A | palmitic acid | NaOH (0.5%) | room temperature | 94.2 | 5.9 | 0 | 85 |
| | B | " | " (1.0%) | " | 93.3 | 6.6 | 0 | 150 |
| | C | " | " (5.0%) | " | 63.2 | 35.3 | 1.5 | 201 |
| Example 8 | A | vitamin E | NaOH (0.1%) | 80°C | 93.5 | 6.5 | 0 | 123 |
| | B | " | " (2.0%) | 80°C | 74.3 | 25.1 | 0.6 | 179 |
| | C | " | " (5.0%) | room temperature | 98.5 | 1.5 | 0 | 205 |
| Example 9 | A | vitamin E | $BF_3$ (0.01%) | room temperature | 86.2 | 13.8 | 0 | 130 |
| | B | " | " (0.1%) | " | 87.5 | 12.5 | 0 | 112 |
| | C | " | " (1.0%) | " | 94.5 | 5.0 | 0.5 | 182 |
| Control | | - | - (-) | - | 44.3 | 39.7 | 16.1 | 174 |
| Example 12 | A | linolic acid | NaOH (0.5%) | room temperature | 72.5 | 27.5 | 0 | 40 |
| | B | " | " (1.0%) | " | 77.7 | 22.2 | 0 | 153 |
| | C | " | " (0.5%) | 80°C | 86.5 | 12.8 | 0.8 | 133 |
| | D | " | " (1.0%) | " | 69.1 | 28.9 | 2.0 | 152 |
| Example 13 | A | linolic acid | $BF_3$ (1.0%) | room temperature | 71.5 | 26.8 | 1.6 | 123 |
| | B | " | " ( " %) | 80°C | 63.0 | 32.0 | 5.0 | 200 |
| Control | | - | - ( - ) | - | 70.2 | 27.0 | 2.8 | 463 |

Example 14

Hollow fibers of regenerated cellulose of cuprammonium were placed in a glass tube, with the ends thereof on one side connected to an aspirator and the ends thereof on the other side immersed in an aqueous 0.5 w/v % NaOH solution. The hollow fibers were filled with the aqueous NaOH solution by virtue of the force of suction generated by the aspirator. After the hollow fibers were filled with the aqueous solution, they were left standing at normal room temperature for 30 minutes. Then, the hollow fibers were emptied of the aqueous NaOH solution. Then, the dioxane solution of 0.5 w/v % of linolic acid macromer obtained in Example 1 was placed in a dialyzer in the same manner as in Example 1 and left standing therein at normal room temperature for 24 hours. The linolic acid macromer solution was discharged and the hollow fibers were washed with dioxane, thoroughly washed with distilled water, and dried with a force current of air at 25° C. To ensure perfect desiccation, the hollow fibers were left standing overnight in an oven at 60° C

A hollow fiber bundle 5 was formed by using 341 hollow fibers of regenerated cellulose of cuprammonium about 200 μm in inside diameter, about 224μm in outside diameter, and 14 cm in available length. This bundle was inserted in a cylinder body 4, with the opposite ends fastened with polyurethane type potting compounds 6, 7, fitted with headers 10, 11 and fixed with fitting caps 12, 13 to give rise to dialyzer (artificial kidney) 1 as illustrated in Fig. 1. In the dialyzer illustrated in Fig. 1, the cylinder body 4 is provided near the opposite ends thereof with an inlet tube 2 and an outlet tube 3 for dialyzing solution. Then, the dialyzer was filled with distilled water and placed in an autoclave and sterilized therein at a temperature of 115° C for 30 minutes.

Evaluation Test 2: Extracorporeal circulation test

A rabbit was fastened on the back on a Kitajima type fixing table. With an electric haircutter, the hair in the region destined to be surgically operated was cut. The freshly bared region of the skin was wiped with a wad of cotton impregnated with alcohol. The skin was cut open with a pair of scissors along the median line extending between the underside of the jaw and the calvicle. Further, the fascia was cut open and the right (left) common carotid artery was peeled off with due attention paid to the safekeeping of the nerves, the ramified blood vessels, and the neighboring tissues. Subsequently, the left(right) anterior facial vein was deeply peeled off similarly attentively. A permanent catheter (produced by Terumo Kabushiki Kaisha and marketed under registered trademark designation of "SURFLO"®) filled with physiological saline water containing 1 IU of heparin per ml and fitted with an injection rubber cap was inserted into the vein and tied fast. The catheter was similarly inserted into the artery and tied fast thereto.

For the rabbit 20 prepared as described above, an experimental circuit was estabilished using the dialyzer obtained in Example 14 and a dialyzer of untreated hollow fibers of regenerated cellulose of cuprammonium having the same membrane surface and serving as control. As illustrated in Fig. 2, a catheter 21 connected to the artery of the rabbit 20 was connected to a pump 22. Further, a chamber 23 and the vein of the rabbit 20 were interconnected with a catheter 25. The pump 22 and the dialyzer 1 were interconnected with a tube 26. This tube 26 communicated with the in 27 side of the manometer. The dialyzer 1 and a chamber 23 communicating with the out 24 side of the manometer were interconnected with a tube 28. The inlet and outlet of the dialyzer for the dialyzing solution were interconnected with a tube 29. The tube 29 was furnished with a pump 30 and, at the same time, immersed in a water bath 31 kept at 37° C. The circuit thus constructed was primed and washed with 100 ml of physiological saline water containing 1 IU of heparin per ml.

The extracorporeal circulation was carried out, with the flow volume of the blood fixed at 10 ml/min. The rabbit was administered with 300 IU of heparin per kg as an anticoagulant 10 minutes before the start of the extracorporeal circulation. After 60 minutes' extracorporeal circulation, the rabbit was additionally administered with 100 IU of heparin per kg. Then, the circulation was continued for two hours. The blood of the rabbit was sampled in a unit amount of 1 ml imediately after start of the circulation and on elapse of 5 minutes, 10 minutes, 15 minutes, 20 minutes, 30 minutes, 45 minutes, 60 minutes, and 120 minutes. The samples were each treated with a physiological saline water containing 1.5% of EDTA-2Na as an anticoagulant and measured with a device (produced by Orth Instrument Corp. and marketed under product code of "ELT-8") to find the number of blood cells. The numbers of white blood cells (WBC), the numbers of platelets (PLT), and the hematocrit values (HCT) found in the test are shown in Tables 2 to 4. Table 2 shows the data obtained in the experimental circuit using a dialyzer packed with hollow fibers of regenerated cellulose of macromer-treated cuprammonium obtained in Example 1, Table 3 the data obtained in the experimental circuit using a dialyzer packed with hollow fibers of regenerated cellulose of untreated cuprammonium used as control, and Table 4 the data obtained in the extracorporeal circulation using the same experimental circuit minus a dialyzer. The numbers of white blood cells and the numbers of platelets were compensated for Ht value in accordance with the following formula and were reported as values in terms of Ht value immediately before start of the circulation.

$$Cx = Co \frac{Htx}{Hto}$$

wherein Cx stands for corrected value, Co for found value. Htx for corrected standard Ht value = initial Ht value, and Hto for Ht value at the time Co value was obtained.

The variation in the number of white blood cells based on these data is shown in the graph of Fig. 3.

Table 2

| Time | WBC | | PLC | | HTC | |
|---|---|---|---|---|---|---|
| | /mm$^3$ | (%) | x10$^4$/mm$^3$ | PIC (%) | % | PIC (%) |
| Start | 9450 | 100 | 34.4 | 100 | 44.2 | 100 |
| 5 min. | 7350 | 79.2 | 33 | 96 | 44.3 | 100.3 |
| 10 min. | 6500 | 69.9 | 31.4 | 92 | 44.1 | 99.9 |
| 15 min. | 6500 | 69 | 31.7 | 91.1 | 44.7 | 101.2 |
| 20 min. | 6650 | 70.9 | 30.5 | 88.6 | 44.3 | 100.4 |
| 30 min. | 7000 | 74.8 | 32.4 | 94.2 | 44.3 | 100.4 |
| 45 min. | 8650 | 91.9 | 32.2 | 92.7 | 44.7 | 101.2 |
| 60 min. | 9150 | 97.8 | 32.5 | 94.1 | 44.4 | 100.6 |
| 120 min. | 9450 | 102 | 31.8 | 94.3 | 43.4 | 98.3 |
| PIC stands for percent initial count. The value reported herein are each an average of numeral values obtained of two test pieces. | | | | | | |

Table 3

| Time | WBC | | PLC | | HTC | |
|---|---|---|---|---|---|---|
| | /mm$^3$ | (%) | x10$^4$/mm$^3$ | PIC (%) | % | PIC (%) |
| Start | 7600 | 100 | 33.2 | 100 | 38.1 | 100 |
| 5 min. | 5000 | 66.2 | 31.8 | 94 | 39.2 | 102.8 |
| 10 min. | 3700 | 48.6 | 29.7 | 87.6 | 39.4 | 103.5 |
| 15 min. | 3400 | 44.2 | 30.4 | 87.2 | 39.8 | 104.4 |
| 20 min. | 3800 | 48.4 | 30 | 85.1 | 40 | 104.9 |
| 30 min. | 4450 | 53 | 36.3 | 104.8 | 41.3 | 108.4 |
| 45 min. | 6300 | 79.2 | 30.5 | 83.9 | 40.3 | 105.7 |
| 60 min. | 7100 | 90.5 | 31.6 | 86.1 | 40.2 | 105.5 |
| 120 min. | 8550 | 114.7 | 28 | 80.3 | 39.1 | 102.7 |
| PIC stands for percent initial count. The value reported herein are each an average of numeral values obtained of two test pieces. | | | | | | |

Table 4

| Time | WBC | | PLC | | HTC | |
|---|---|---|---|---|---|---|
| | /mm$^3$ | (%) | x10$^4$/mm$^3$ | PIC (%) | % | PIC (%) |
| Start | 5367 | 100 | 42.2 | 100 | 39.6 | 100 |
| 5 min. | 5133 | 97.2 | 42.1 | 101.1 | 39 | 99 |
| 10 min. | 5000 | 93.1 | 42 | 99.6 | 39.5 | 100.2 |
| 15 min. | 5133 | 95 | 43.4 | 103.6 | 39.4 | 99.9 |
| 20 min. | 4633 | 86.3 | 42.9 | 102.2 | 39.4 | 99.8 |
| 30 min. | 4600 | 84.3 | 43.5 | 103.4 | 39.5 | 100.1 |
| 45 min. | 4600 | 85.7 | 41.8 | 100.9 | 38.9 | 98.4 |
| 60 min. | 4767 | 89 | 40.2 | 98.1 | 38.6 | 97.8 |
| 120 min. | 5833 | 112.5 | 38.9 | 96.3 | 37.9 | 95.8 |
| PIC stands for percent initial count. The value reported herein are each an average of numeral valued obtained of three test pieces. | | | | | | |

(Industrial Applicability)

This invention, as described above, is directed to a medical material characterized by possessing on the surface of a substrate made of a polymer possessing functional group-containing repeating units a graft polymer formed by causing a macromer possessing at one of the opposite terminals thereof a fat-soluble vitamin, a saturated fatty acid, or an unsaturated fatty acid residue to be bonded at the remaining reactive terminal thereof to the aforementioned functional group of the polymer existing on the surface of the substrate. This medical material, therefore, is excellent in respect that it is endowed, without a sacrifice of the highly desirable bulk property inherent in the substrate, with a surface behavior exhibiting high biocompatibility stably for a long time. It can be utilized advantageously in such applications as artificial internal organs and artificial blood vessels. Since the graft chains are uniform and prominent, the length and mobility of graft chains can be controlled with ease. The fat-soluble vitamin, unsaturated fatty acid, or saturated fatty acid which is bonded to the leading ends of these graft chains contributes to further enhancing the biocompatibility of the produced medical material. The medical material of the present invention, therefore, contributes immensely to various field of medicine and medical engineering. The medical material of this invention is enabled to manifest the excellent characteristic properties thereof to better advantage when the functional group is one member selected from the class consisting of hydroxyl group, amino group, and carboxyl group, and preferably is hydroxyl group, the polymer is a regenerated cellulose or a cellulose derivative, the fat-soluble vitamin is one member selected from the group consisting of vitamin A, vitamin D, vitamin E, vitamin K, and ubiquinone, and preferably is vitamin E, the saturated fatty acid is one member selected from the group consisting of laurylic acid, myristic acid, pentadylic acid, palmitic acid, and stearic acid, and preferably myristic acid or palmitic acid, the unsaturated fatty acid is one member selected from the group consisting of elaidic acid, oleic acid, linolic acid, linolenic acid, arachidonic acid, and eicosapentaeic acid, and preferably is linolic acid or linolenic acid, and the macromer possesses a linear diglycidyl ether derivative such as 1,4-butane diol diglycidyl ether possessing a fat-soluble vitamin, saturated fatty acid, or unsaturated fatty acid residue and/or an alkylene glycol skeleton, more desirably an alkylene glycol skeleton having a polymerization degree in the range of 1 to 100, and most desirably polyethylene glycol or polypropylene glycol skeleton.

The present invention is further directed to a method for the production of a medical material characterized by (a) forming a macromer possessing at one of the opposite terminals thereof fat-soluble vitamin, a saturated fatty acid, or an unsaturated fatty acid residue and (b) causing the other reactive terminal of the macromer to be bonded, on a substrate made of a polymer possessing functional group-containing repeating units, to the functional group of the polymer existing on the surface of the substrate. The medical material of the present invention which possesses such highly desirable properties as described above can be produced by a simple procedure. It is also advantageous economically because the method has no use for any special device. Further, the method of this invention for the production of a medical matreial suits utiliy in a very wide range of applications because it is capable of imparting a surface behavior rich in biocompatibility to a polymer possessing a functional group. The method of this invention is utilized advantageously in

the formation of a macromer possessing an epoxy group as the other reactive terminal because this method is capable of further activating the reaction of the macromer with the functional group on the surface of the substrte. The method of this invention permits a macromer possessing highly desirable properties to be produced with increased ease because the process (a) for the formation of the macromer is carried out by selectively causing the reactive group at one of the opposite terminals of a macromer precursor possessing reactive groups on each at the opposite terminals thereof (such as, for example, a macromer precursor possessing diepoxide, preferably a linear diglycidyl ether such as 1,4-butane diol diglycidyl ether and/or an alkylene glycol skeleton) to react with the functional group such as hydroxyl group or carboxyl group in the fat-soluble vitamin, saturated fatty acid, or unsaturated fatty acid. Further in the process (b) for the bonding of the macromer to the surface of the substrate, the bonding reaction involved therein proceeds more advantageously and the graft chains can be joined uniformly only to the surface of the substrate when this bonding is effected by causing the macromer to come into contact in a liquid state or gaseous state with the surface of the substrate thereby allowing the other reactive terminal of the macromer to react with the functional group of the polymer other than those at the opposite terminals thereof present on the surface of the substrate and when, in the embodiment having an epoxy group as the reactive terminal of the macromer and an OH group as the functional group of the substrate, the bonding is effected by causing the macromer to come into contact in a liquid state with the surface of the substrate in the presence of a Friedel-Crafts type catalyst or an alkali catalyst thereby enabling the reactive epoxy terminal of the macromer to react with the functional OH group on the surface of the substrate. Further when, in the aforementioned embodiment, the Friedel-Crafts type catalyst is boron trifluoride or the alkali catalyst is sodium hydroxide or potassium hydroxide and the solvent used therein is one member selected from the group consisting of dioxane, acetone, or methylethyl ketone, the reaction proceeds more quickly and advantageously. Since the catalyst and the solvent can be readily removed by washing,the produced medical material also excels in safety. Thus, the method of the present invention brings about highly desirable results.

## Claims

1. A medical material characterized by possessing on the surface of a substrate made of a polymer possessing hydroxyl group, amino group or carboxyl group containing repeating units a graft polymer formed by causing a macromer possessing at one of the opposite terminals thereof a fat-soluble vitamin, a saturated fatty acid, or an unsaturated fatty acid residue to be bonded at the remaining reactive terminal thereof being an epoxy group to said hydroxyl-, amino- or carboxyl group of said polymer existing on said surface of said substrate.

2. A medical material according to Claim 1, wherein said functional group is a hydroxyl group.

3. A medical material according to Claim 2, wherein said polymer is regenerated cellulose or a cellulose derivative.

4. A medical material according to any of Claims 1 to 3, wherein said fat-soluble vitamin is selected from the group consisting of vitamin A, vitamin D, vitamin E, vitamin K, and ubiquinone.

5. A medical material according to Claim 4, wherein said fat-soluble vitamin is vitamin E.

6. A medical material according to any of Claims 1 to 3, wherein said saturated fatty acid is selected from the group consisting of laurylic acid, myristic acid, pentadecylic acid, palmitic acid, and stearic acid.

7. A medical material according to Claim 6, wherein said saturated fatty acid is myristic acid.

8. A medical material according to Claim 6, wherein said saturated fatty acid is palmitic acid.

9. A medical material according to any of Claims 1 to 3, wherein said unsaturated fatty acid is selected from the group consisting of elaidic acid, oleic acid, linolic acid, linolenic acid, arachidonic acid, and eicosapentaeic acid.

10. A medical material according to Claim 9, wherein said unsaturated fatty acid is linolic acid.

11. A medical material according to Claim 9, wherein said unsaturated fatty acid is linolenic acid.

12. A medical material according to Claim 1 , wherein said macromer is formed of a linear diglycidyl ether derivative possessing at one of the opposite terminals thereof a fat-soluble vitamin, saturated fatty acid, or unsaturated fatty acid residue.

13. A medical material according to Claim 12, wherein said linear diglycidyl ether is 1,4-butane diol diglycidyl ether.

14. A medical material according to any of Claims 1 to 11, wherein said macromer is formed of at least one alkylene glycol skeleton possessing at one of the opposite terminals thereof a fat-soluble vitamin, saturated fatty acid, or unsaturated fatty acid residue.

15. A medical material according to Claim 14, wherein said alkylene glycol has a polymerization degree in the range of 1 to 100.

16. A medical material according to any of Claims 14 to 15, wherein said alkylene glycol is polyethylene glycol.

17. A medical material according to any of Claims 14 to 15, wherein said alkylene glycol is polypropylene glycol.

18. A method for the production of a medical material characterized by

(a) forming a macromer possessing at one of the opposite terminals thereof a fat-soluble vitamin, a saturated fatty acid, or an unsaturated fatty acid residue and
(b) causing the other reactive terminal of said macromer being an epoxy group to be bonded, on a substrate made of a polymer possessing hydroxyl-, amino- or carboxyl group-containing repeating units, to said hydroxyl-, amino- or carboxyl-group of said polymer existing on the surface of said substrate.

19. A method for the production of a medical material according to Claim 18, wherein said macromer is formed by causing the reactive group at one of the opposite terminals thereof to react selectively with the functional group of said fat-soluble vitamin, saturated fatty acid, or unsaturated fatty acid.

20. A method for the production of a medical material according to Claim 19, wherein said macromer precursor is diepoxide.

21. A method for the production of a medical material according to Claim 20, wherein said diepoxide is a linear diglycidyl ether.

22. A method for the production of a medical material according to Claim 21, wherein said linear diglycidyl ether is 1,4-butane diol diglycidyl ether.

23. A method for the production of a medical material according to any of Claims 18 to 21, wherein said macromer precursor possesses at least one alkylene glycol skeleton.

24. A method for the production of a medical material according to any of Claims 18 to 23, wherein the functional group of said fat-soluble vitamin, saturated fatty acid, or unsaturated fatty acid is a carboxyl group.

25. A method for the production of a medical material according to any of Claims 18 to 23, wherein the functional group of said fat-soluble vitamin is a hydroxyl group.

26. A method for the production of a medical material according to any of Claims 18 to 25, wherein said macromer is bonded to the surface of said substrate by bringing said macromer into contact in a liquid state or a gaseous state with the surface of said substrate thereby allowing the functional group on the surface of said substrate to react with the other reactive terminal of said macromer.

27. A method for the production of a medical material according to any of Claims 18 to 26, wherein said macromer is bonded to the surface of said substrate by bringing said macromer into contact in a liquid state with the surface of said substrate possessing a functional OH group in the presence of a Friedel-Crafts type catalyst or an alkali catalyst thereby enabling the reactive epoxy terminal of said macromer to react with the functional OH group on the surface of said substrate.

28. A method for the production of a medical material according to Claim 27, wherein said Friedel-Crafts type catalyst is boron trifluoride.

29. A method for the production of a medical material according to Claim 27, wherein said alkali catalyst is sodium hydroxide or potassium hydroxide.

30. A method for the production of a medical material according to any of Claims 26 to 29, wherein one member

selected from the group consisting of dioxane, acetone, and methylethyl ketone is used as a solvent.

**Patentansprüche**

1. Medizinisches Material, dadurch gekennzeichnet, daß es auf der Oberfläche eines aus einem Polymer mit hydroxyl-, amino- oder carboxylgruppenhaltigen wiederkehrenden Einheiten bestehenden Substrats ein Pfropfpolymer aufweist, das dadurch entstanden ist, daß ein Makromer mit einem fettlöslichen Vitamin-, einem gesättigten Fettsäure- oder einem ungesättigten Fettsäurerest an einem seiner gegenüberliegenden Enden über sein aus einer Epoxygruppe bestehendes restliches reaktionsfähiges Ende an die Hydroxyl-, Amino- oder Carboxylgruppe des auf der Oberfläche des Substrats befindlichen Polymers gebunden wurde.

2. Medizinisches Material nach Anspruch 1, dadurch gekennzeichnet, daß die funktionelle Gruppe aus einer Hydroxylgruppe besteht.

3. Medizinisches Material nach Anspruch 2, dadurch gekennzeichnet, daß das Polymer aus regenerierter Cellulose oder einem Cellulosederivat besteht.

4. Medizinisches Material nach einem der Ansprüche 1 bis 3, dadurch gekennzeichnet, daß das fettlösliche Vitamin aus der Gruppe Vitamin A, Vitamin D, Vitamin E, Vitamin K und Ubichinon ausgewählt ist.

5. Medizinisches Material nach Anspruch 4, dadurch gekennzeichnet, daß das fettlösliche Vitamin aus Vitamin E besteht.

6. Medizinisches Material nach einem der Ansprüche 1 bis 3, dadurch gekennzeichnet, daß die gesättigte Fettsäure aus der Gruppe Laurylsäure, Myristinsäure, Pentadecansäure, Palmitinsäure und Stearinsäure ausgewählt ist.

7. Medizinisches Material nach Anspruch 6, dadurch gekennzeichnet, daß die gesättigte Fettsäure aus Myristinsäure besteht.

8. Medizinisches Material nach Anspruch 6, dadurch gekennzeichnet, daß die gesättigte Fettsäure aus Palmitinsäure besteht.

9. Medizinisches Material nach einem der Ansprüche 1 bis 3, dadurch gekennzeichnet, daß die ungesättigte Fettsäure aus der Gruppe Elaidinsäure, Ölsäure, Linolsäure, Linolensäure, Arachidonsäure und Eicosapentaensäure ausgewählt ist.

10. Medizinisches Material nach Anspruch 9, dadurch gekennzeichnet, daß die ungesättigte Fettsäure aus Linolsäure besteht.

11. Medizinisches Material nach Anspruch 9, dadurch gekennzeichnet, daß die ungesättigte Fettsäure aus Linolensäure besteht.

12. Medizinisches Material nach Anspruch 11, dadurch gekennzeichnet, daß das Makromer aus einem linearen Diglycidyletherderivat mit einem fettlöslichen Vitamin-, gesättigten Fettsäure- oder ungesättigten Fettsäurerest an einem seiner gegenüberliegenden Enden gebildet ist.

13. Medizinisches Material nach Anspruch 12, dadurch gekennzeichnet, daß der lineare Diglycidylether aus 1,4-Butandioldiglycidylether besteht.

14. Medizinisches Material nach einem der Ansprüche 1 bis 11, dadurch gekennzeichnet, daß das Makromer aus mindestens einem Alkylenglykolskelett mit einem fettlöslichen Vitamin-, gesättigten Fettsäure- oder ungesättigten Fettsäurerest an einem seiner gegenüberliegenden Enden gebildet ist.

15. Medizinisches Material nach Anspruch 14, dadurch gekennzeichnet, daß das Alkylenglykol einen Polymerisationsgrad im Bereich von 1 bis 100 aufweist.

16. Medizinisches Material nach einem der Ansprüche 14 bis 15, dadurch gekennzeichnet, daß das Alkylenglykol aus Polyethylenglykol besteht.

**17.** Medizinisches Material nach einem der Ansprüche 14 bis 15, dadurch gekennzeichnet, daß das Alkylenglykol aus Polypropylenglykol besteht.

**18.** Verfahren zur Herstellung eines medizinischen Materials, gekennzeichnet durch

a) Ausbildung eines Makromers mit einem fettlöslichen Vitamin-, gesättigten Fettsäure- oder ungesättigten Fettsäurerest an einem seiner gegenüberliegenden Enden und

b) Herbeiführung einer Bindung zwischen dem aus einer Epoxygruppe bestehenden, anderen reaktionsfähigen Ende des Makromers und den auf der Oberfläche eines Substrats aus einem Polymer mit hydroxyl-, amino- oder carboxylgruppenhaltigen wiederkehrenden Einheiten befindlichen Hydroxyl-, Amino- oder Carboxylgruppen des Polymers.

**19.** Verfahren zur Herstellung eines medizinischen Materials nach Anspruch 18, dadurch gekennzeichnet, daß das Makromer durch selektives Reagierenlassen der reaktionsfähigen Gruppe an einem seiner gegenüberliegenden Enden mit der funktionellen Gruppe des fettlöslichen Vitamins, der gesättigten Fettsäure oder der ungesättigten Fettsäure gebildet wird.

**20.** Verfahren zur Herstellung eines medizinischen Materials nach Anspruch 19, dadurch gekennzeichnet, daß der Makromervorläufer aus einem Diepoxid besteht.

**21.** Verfahren zur Herstellung eines medizinischen Materials nach Anspruch 20, dadurch gekennzeichnet, daß das Diepoxid aus einem linearen Diglycidylether besteht.

**22.** Verfahren zur Herstellung eines medizinischen Materials nach Anspruch 21, dadurch gekennzeichnet, daß der lineare Diglycidylether aus 1,4-Butandioldiglycidylether besteht.

**23.** Verfahren zur Herstellung eines medizinischen Materials nach einem der Ansprüche 18 bis 21, dadurch gekennzeichnet, daß der Makromervorläufer zumindest ein Alkylenglykolskelett besitzt.

**24.** Verfahren zur Herstellung eines medizinischen Materials nach einem der Ansprüche 18 bis 23, dadurch gekennzeichnet, daß die funktionelle Gruppe desfettlöslichen Vitamins, der gesättigten Fettsäure oder der ungesättigten Fettsäure aus einer Carboxylgruppe besteht.

**25.** Verfahren zur Herstellung eines medizinischen Materials nach einem der Ansprüche 18 bis 23, dadurch gekennzeichnet, daß die funktionelle Gruppe des fettlöslichen Vitamins aus einer Hydroxylgruppe besteht.

**26.** Verfahren zur Herstellung eines medizinischen Materials nach einem der Ansprüche 18 bis 25, dadurch gekennzeichnet, daß das Makromer an die Oberfläche des Substrats gebunden wird, indem das Makromer in flüssigem oder gasförmigem Zustand mit der Oberfläche des Substrats zur Reaktion der funktionellen Gruppe auf der Oberfläche des Substrats mit dem anderen reaktionsfähigen Ende des Makromers in Berührung gebracht wird.

**27.** Verfahren zur Herstellung eines medizinischen Materials nach einem der Ansprüche 18 bis 26, dadurch gekennzeichnet, daß das Makromer an die Oberfläche des Substrats gebunden wird, indem das Makromer in flüssigem Zustand und in Gegenwart eines Katalysators vom Friedel-Crafts-Typ oder eines Alkalikatalysators mit der Oberfläche des eine funktionelle OH-Gruppe enthaltenden Substrats zur Reaktion des reaktionsfähigen Epoxyendes des Makromers mit der funktionellen OH-Gruppe auf der Oberfläche des Substrats in Berührung gebracht wird.

**28.** Verfahren zur Herstellung eines medizinischen Materials nach Anspruch 27, dadurch gekennzeichnet, daß der Katalysator vom Friedel-Crafts-Typ aus Bortrifluorid besteht.

**29.** Verfahren zur Herstellung eines medizinischen Materials nach Anspruch 27, dadurch gekennzeichnet, daß der Alkalikatalysator aus Natriumhydroxid oder Kaliumhydroxid besteht.

**30.** Verfahren zur Herstellung eines medizinischen Materials nach einem der Ansprüche 26 bis 29, dadurch gekennzeichnet, daß als Lösungsmittel eines aus der Gruppe Dioxan, Aceton und Methylethylketon verwendet wird.

**Revendications**

1. Un matériau médical caractérisé en ce qu'il possède, sur la surface d'un support fait d'un polymère possédant des motifs récurrents contenant un coupe hydroxyle, un groupe amino ou un groupe carboxyle, un polymère greffé formé en reliant un macromère, possédant à l'une de ses extrémités opposées un résidu de vitamine soluble dans les graisses, un résidu d'acide gras saturé ou un résidu d'acide gras insaturé, par son extrémité réactive restante qui est un groupe époxy, audit groupe hydroxyle, amino ou carboxyle dudit polymère existant sur ladite surface dudit support.

2. Un matériau médical selon la revendication 1, dans lequel ledit groupe fonctionnel est un groupe hydroxyle.

3. Un matériau médical selon la revendication 2, dans lequel ledit polymère est la cellulose régénérée ou un dérivé de cellulose.

4. Un matériau médical selon l'une quelconque des revendications 1 à 3, dans lequel ladite vitamine soluble dans les graisses est choisie parmi la vitamine A, la vitamine D, la vitamine K et l'ubiquinone.

5. Un matériau médical selon la revendication 4, dans lequel ladite vitamine soluble dans les graisses est la vitamine E.

6. Un matériau médical selon l'une quelconque des revendications 1 à 3, dans lequel ledit acide gras saturé est choisi parmi l'acide laurique, l'acide myristique, l'acide pentadécylique, l'acide palmitique et l'acide stéarique.

7. Un matériau médical selon la revendication 6, dans lequel ledit acide gras saturé est l'acide myristique.

8. Un matériau médical selon la revendication 6, dans lequel ledit acide gras saturé est l'acide palmitique.

9. Un matériau médical selon l'une quelconque des revendications 1 à 3, dans lequel ledit acide gras insaturé est choisi parmi l'acide élaïdique, l'acide oléique, l'acide linolique, l'acide linolénique, l'acide arachidonique et l'acide eicosapentaénique.

10. Un matériau médical selon la revendication 9, dans lequel ledit acide gras insaturé est l'acide linolique.

11. Un matériau médical selon la revendication 9, dans lequel ledit acide gras insaturé est l'acide linolénique.

12. Un matériau médical selon la revendication 1, dans lequel ledit macromère est formé d'un dérivé d'éther diglycidylique linéaire possédant à l'une de ses extrémités opposées un résidu de vitamine soluble dans les graisses, un résidu d'acide gras saturé ou un résidu d'acide gras insaturé.

13. Un matériau médical selon la revendication 12, dans lequel ledit éther diglycidylique linéaire est l'éther diglycidylique de 1,4-butanediol.

14. Un matériau médical selon l'une quelconque des revendications 1 à 11, dans lequel ledit macromère est formé d'au moins un squelette d'alkylèneglycol possédant à l'une de ses extrémités opposées un résidu de vitamine soluble dans les graisses, un résidu d'acide gras saturé ou un résidu d'acide gras insaturé.

15. Un matériau médical selon la revendication 14, dans lequel ledit alkylèneglycol a un degré de polymérisation dans la gamme de 1 à 100.

16. Un matériau médical selon l'une quelconque des revendications 14 à 15, dans lequel ledit alkylèneglycol est un polyéthylèneglycol.

17. Un matériau médical selon l'une quelconque des revendications 14 à 15, dans lequel ledit alkylèneglycol est un polypropylèneglycol.

18. Un procédé pour la production d'un matériau médical, caractérisé par les étapes suivantes :

a) on forme un macromère possédant à l'une de ses extrémités opposées un résidu de vitamine soluble dans les graisses, un résidu d'acide gras saturé ou un résidu d'acide gras insaturé et

b) on relie l'autre extrémité réactive dudit macromère, qui est un groupe époxy, sur un support fait d'un polymère possédant des motifs récurrents contenant un groupe hydroxyle, amino ou carboxyle, audit groupe hydroxyle, amino ou carboxyle dudit polymère existant sur la surface dudit support.

**19.** Un procédé pour la production d'un matériau médical selon la revendication 18, dans lequel ledit macromère est formé en faisant réagir sélectivement le groupe réactif à l'une de ses extrémités opposées avec le groupe fonctionnel de ladite vitamine soluble dans les graisses, dudit acide gras saturé ou dudit acide gras insaturé.

**20.** Un procédé pour la production d'un matériau médical selon la revendication 19, dans lequel ledit précurseur du macromère est un diépoxyde.

**21.** Un procédé pour la production d'un matériau médical selon la revendication 20, dans lequel ledit diépoxyde est un éther diglycidylique linéaire.

**22.** Un procédé pour la production d'un matériau médical selon la revendication 21, dans lequel ledit éther diglycidylique linéaire est l'éther diglycidylique de 1,4-butanediol.

**23.** Un procédé pour la production d'un matériau médical selon l'une quelconque des revendications 18 à 21, dans lequel ledit précurseur du macromère possède au moins un squelette d'alkylèneglycol.

**24.** Un procédé pour la production d'un matériau médical selon l'une quelconque des revendications 18 à 23, dans lequel ledit groupe fonctionnel de ladite vitamine soluble dans les graisses, dudit acide gras saturé ou dudit acide gras insaturé est un groupe carboxyle.

**25.** Un procédé pour la production d'un matériau médical selon l'une quelconque des revendications 18 à 23, dans lequel le groupe fonctionnel de ladite vitamine soluble dans les graisses est un groupe hydroxyle.

**26.** Un procédé pour la production d'un matériau médical selon l'une quelconque des revendications 18 à 25, dans lequel ledit macromère est lié à la surface dudit support par mise en contact dudit macromère à l'état liquide ou à l'état gazeux avec la surface dudit support, en faisant ainsi réagir le groupe fonctionnel à la surface dudit support avec l'autre extrémité réactive dudit macromère.

**27.** Un procédé pour la production d'un matériau médical selon l'une quelconque des revendications 18 à 26, dans lequel ledit macromère est lié à la surface dudit support par mise en contact dudit macromère à l'état liquide avec la surface dudit support possédant un groupe fonctionnel OH en présence d'un catalyseur du type Friedel-Crafts ou d'un catalyseur alcalin, en faisant ainsi réagir le groupe époxy terminal réactif dudit macromère avec le groupe OH fonctionnel à la surface dudit support.

**28.** Un procédé pour la production d'un matériau médical selon la revendication 27, dans lequel le catalyseur du type Friedel-Crafts est le trifluorure de bore.

**29.** Un procédé pour la production d'un matériau médical selon la revendication 27, dans lequel ledit catalyseur alcalin est l'hydroxyde de sodium ou l'hydroxyde de potassium.

**30.** Un procédé pour la production d'un matériau médical selon l'une quelconque des revendications 26 à 29, dans lequel on utilise comme solvant un composé choisi parmi le dioxanne, l'acétone et la méthyléthylcétone.

FIG. 1

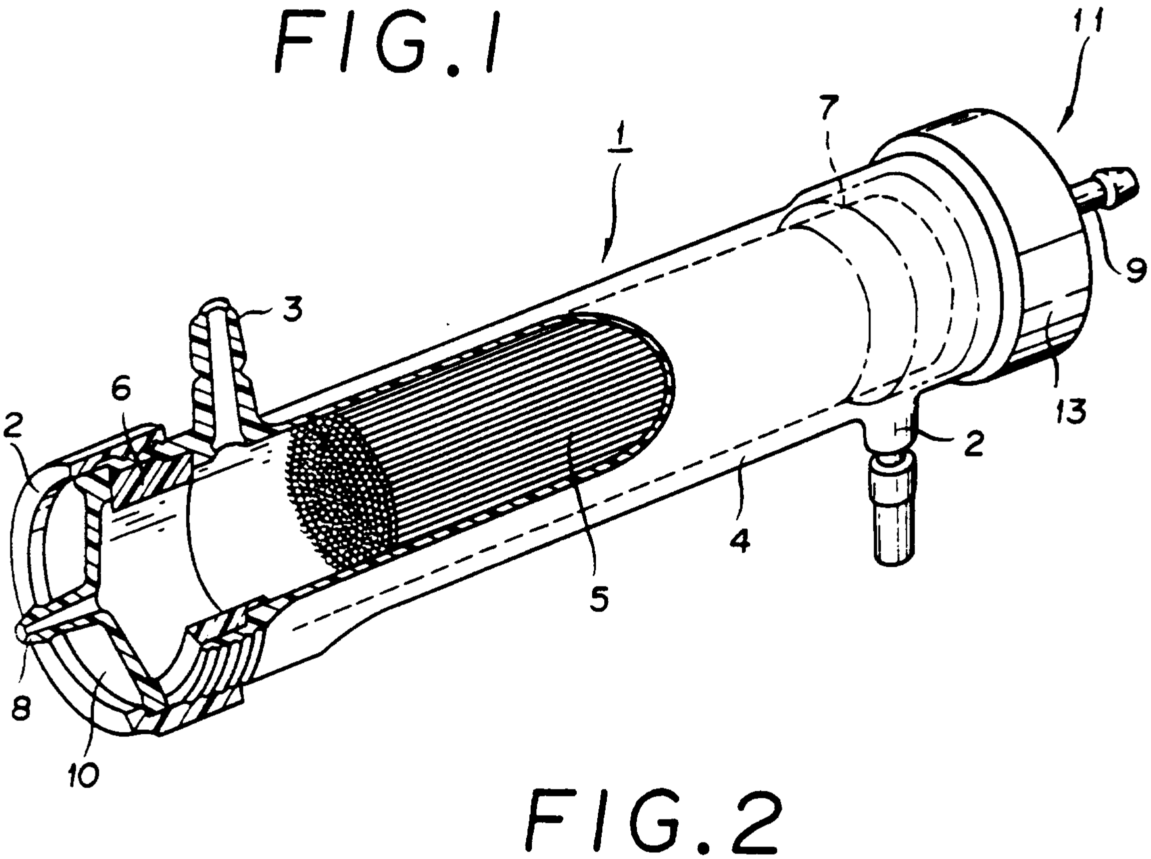


FIG. 2

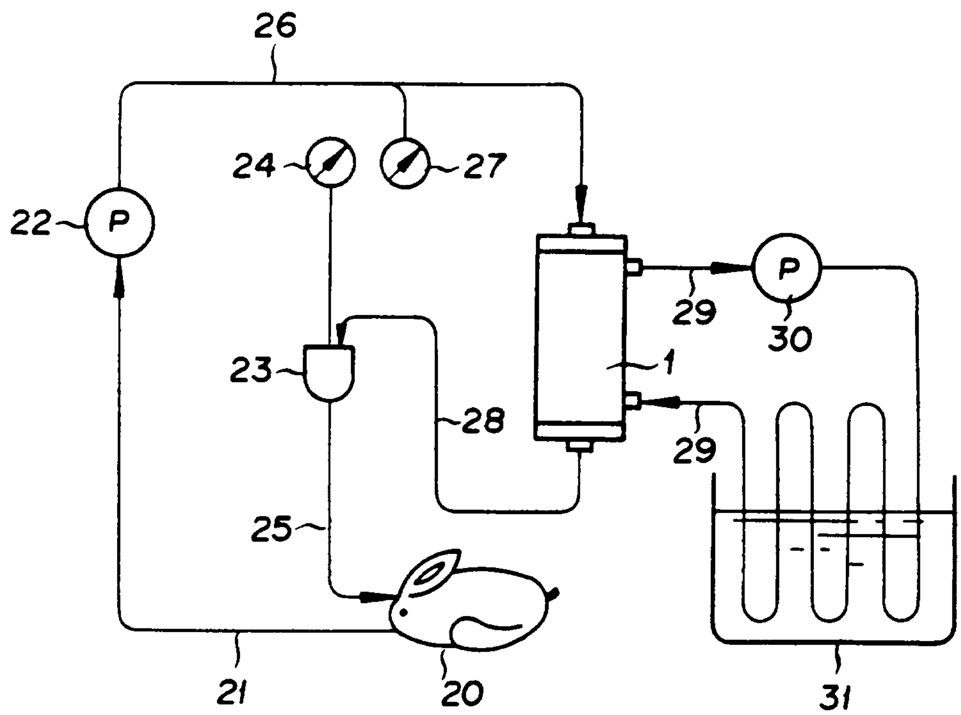

FIG. 3

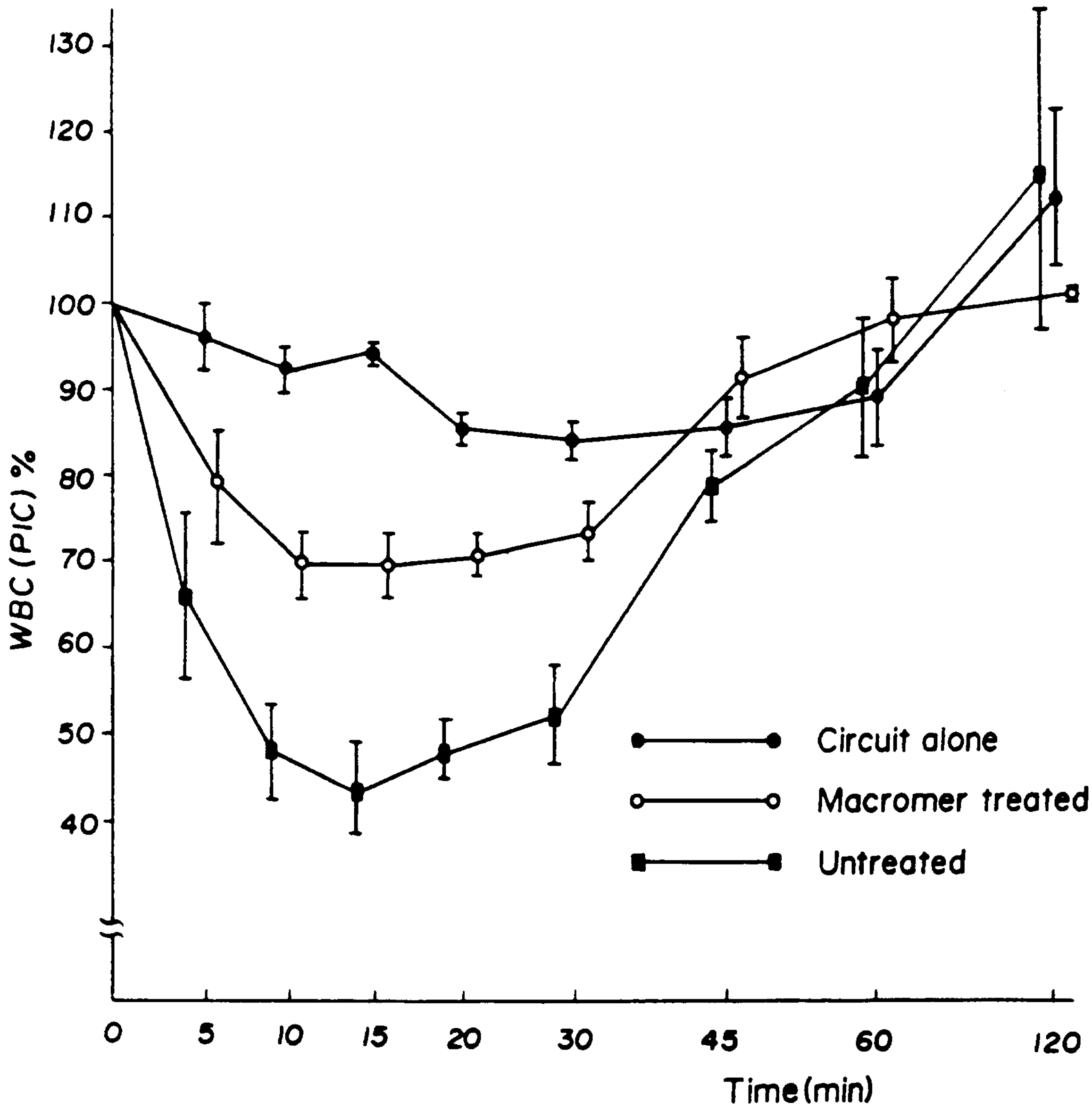
130
120
110
100
90
80
70
60
50
40
WBC (PIC) %
0
5
10
15
20
30
45
60
120
Time (min)
Circuit alone
Macromer treated
Untreated